# EUROPEAN PATENT APPLICATION

(11) **EP 2 221 010 A1**
(43) Date of publication of application: **25.08.2010**
(21) Application number: 10153842.9
(22) Date of filing: 17.02.2010
(51) Int. Cl.: A61B 17/128, A61B 17/122

(54) **Clip package, multiple clip applicator system, and prevention device for preventing mismatch**

(30) Priority: 24.02.2009 JP 2009040255
(71) Applicant: FUJIFILM Corporation, Minato-ku Tokyo (JP)
(72) Inventor: Matsuoka, Yoshiaki, Saitama (JP); Itoh, Koji, Saitama (JP); Oosako, Ryousuke, Saitama (JP); Iida, Takayuki, Saitama (JP)
(74) Representative: Klunker . Schmitt-Nilson . Hirsch

(57) **Abstract**

A multiple clip applicator system for tissue clamping of hemostasis includes clip devices (21, 21A-21C, 21S, 21M, 21L) of a train and a flexible sheath (16). To load the flexible sheath with clip devices, a coupling device (15) is used. A shaft head (52, 52S, 52M, 52L, 140, 140S, 140M, 140L) is positioned on an operating wire (17) in the flexible sheath. A receiving opening (82, 83S, 83M, 83L, 142S, 142M, 142L) is formed in the coupling device, for receiving insertion of the shaft head, for mating by press-fit with the fastening clip device (23). The shaft head is a selected one of plural shaft heads which are different in a shape according to the plural multiple clip assemblies (13). The receiving opening is a selected one of plural receiving openings having a shape according to corresponding shapes of the plural shaft heads, and adapted to preventing a second shaft head from entry in a first receiving opening and preventing a first shaft head from entry in a second receiving opening.

## Description

### BACKGROUND OF THE INVENTION

### 1. Field of the Invention

The present invention relates to a clip package, multiple clip applicator system, and prevention device for preventing mismatch. More particularly, the present invention relates to a clip package which includes a train of plural clip devices and in which mismatch of a multiple clip assembly on a handle device is prevented, and multiple clip applicator system, and prevention device for preventing mismatch.

### 2. Description Related to the Prior Art

An applied use of an endoscope is known in the field of medicine, and used for treatment by entry of a long medical instrument in a patient's body. Tissue clamping is known as treatment of affected tissue with the endoscope, in which affected tissue in the body is clamped by a clip device, for example hemostasis. A pair of arms are included in the clip device, constituted by bending a strip of metal, and shiftable between open and closed positions. In a free state, the arms become open with the resiliency of the strip. Claws are formed at distal ends of the arms, for mesh with one another when closed.

In a clip device applicator system, a handle device includes a flexible sheath and an operating wire. The flexible sheath is loaded with the clip device. The operating wire is coupled with a proximal end of the clip device within the flexible sheath. The flexible sheath with the clip device is entered in the body through an instrument channel which is defined within the endoscope. When the operating wire is pulled, the clip device is moved to protrude from a distal opening of the flexible sheath. The arms are opened in the clip device with their resiliency. When the claws of the clip device is pressed on the affected tissue, the operating wire is pulled. The distal opening of the flexible sheath or a tubular shell about the clip device closes the clip device, which is separated from the operating wire.

U.S.P. No. 6,814,742 (corresponding to JP-A 2007-222649) discloses a clip device contained in a housing for loading a flexible sheath with the clip device. To set the clip device on the flexible sheath, a distal end of the flexible sheath is inserted in a hole in the housing. A shaft head for hooking or press-fit at a distal end of an operating wire is mated with the clip device, before the operating wire is introduced in the flexible sheath.

It is preferable for the clip device to have as small a size as possible because of small influence to a body. However, tissue clamping may be impossible with a small size of the clip device as there are various sizes of affected tissue. Thus, a plurality of types of clip devices with different sizes are prepared in a system of a known clip applicator. An operator or doctor can select one of the clip devices with a suitable size according to the affected tissue or the like. Note that the size of the clip devices means a length of arms, or a width of opening of the arms. Examples of the sizes is a standard size, long size and short size.

The well-known one-shot type of the clip applicator (See U.S.P. No. 6,814,742 corresponding to JP-A 2007-222649) can load only one clip device. It is necessary to pull the clip applicator out of an endoscope at each one of the tissue clamping, load another clip device, and reinsert the clip applicator in the endoscope. This requires very complicated operation. In view of such difficulties, there have been suggestions of a multiple clip applicator which enables successive operation of the tissue clamping. In U.S.P. No. 7,081,121 (corresponding to JP-A 2002-272751), the multiple clip applicator loads the flexible sheath with a plurality of the clip devices connected in a sequential manner by use of claws. The operating wire is coupled with a final one of the clip devices. The operating wire is moved to advance the clip devices through a distal opening of the flexible sheath one after another. A first one of the clip devices operates for the tissue clamping of the affected tissue before the connection is released.

Also, a multiple clip package has been suggested in the field of tissue clamping for the purpose of easily loading the flexible sheath with a plurality of the clip devices. The clip package includes a multiple clip assembly or clip train, which includes a plurality of the clip devices connected to one another, and is contained in the housing. The clip package operates for loading the flexible sheath with the multiple clip assembly from the housing.

In the multiple clip applicator, a moving amount or stroke of the operating wire is different according to a sequence or positions of connecting the clip devices within the multiple clip assembly for the purpose of advancing the clip devices through a distal opening of the flexible sheath. For example, a handle device is constructed to advance the clip devices by moving the operating wire in a distal direction from its home position. The moving amount or stroke of the operating wire is greater for a final one of the clip devices than a first one of the clip devices. Also, the moving amount or stroke of the operating wire is influenced by a size of the clip devices, and differs between the types of the clip devices with the short, medium and long sizes.

In the clip applicator of the one-shot type, a doctor or operator moves the operating wire by observing an image obtained by the endoscope. However, the moving amount or stroke of the operating wire for the multiple clip applicator is different for each of the clip devices for the purpose of the tissue clamping. Much care must be taken for operating the multiple clip applicator. In consideration of this problem, it is necessary to change over the moving amount or stroke of the operating wire by use of the handle device according to the position of the clip devices in the multiple clip assembly. Also, the handle device of a plurality of types may be conceivable for the multi size system with the short, medium and long sizes of the clip devices.

When plural types of multiple clip assemblies are combined with plural types of handle devices, there occurs a problem of mismatch in that one of the handle device is loaded with an incorrect one of the multiple clip assembly. In such a situation, the distance of advancing the clip device relative to the flexible sheath is insufficient or excessive. Failure occurs in that the arms of the clip device do not open suitably, or that the clip device is disconnected unsuitably before the tissue clamping.

As the clip devices have as small a length as several millimeters, it is highly difficult to discern the size of the clip devices by observing the appearance. If colors of the housing are predetermined distinctly for the types of the clip devices, prevention of mismatch cannot be complete. Types of the clip devices may be indicated on a surface of the housing and the handle device, but with a limit for effectively preventing mismatch. There is no suggestion of prevention of mismatch in the documents of U.S.P. No. 6,814,742 (corresponding to JP-A 2007-222649) and U.S.P. No. 7,081,121 (corresponding to JP-A 2002-272751).

### SUMMARY OF THE INVENTION

In view of the foregoing problems, an object of the present invention is to provide a clip package which includes a train of plural clip devices and in which mismatch of a multiple clip assembly on a handle device is prevented, and multiple clip applicator system, and prevention device for preventing mismatch.

In order to achieve the above and other objects and advantages of this invention, a clip package is provided, and includes a multiple clip assembly having plural clip devices connected with one another in a train by use of claws positioned at distal ends of openable arms, a fastening clip device connected with a final one of the clip devices, and a fitting connector, disposed on the fastening clip device, for mating with a shaft head in a direction transverse to a direction of the train of the clip devices at a distal end of an operating wire for operating the clip devices. A housing contains the multiple clip assembly. There is a coupling device including a stage portion for introducing the multiple clip assembly from the housing, to enable coupling with a flexible sheath through which the operating wire extends. A receiving opening has a form slightly larger than a profile of the shaft head and a portion of the operating wire as viewed in the transverse direction, for receiving insertion of the shaft head and the portion of the operating wire to mate the shaft head with the fitting connector on the stage portion. The coupling device operates upon pull of the operating wire after mating the shaft head with the fitting connector, to enter the multiple clip assembly from the stage portion into the flexible sheath in a proximal direction thereof.

Also, a multiple clip applicator system is provided, and includes a multiple clip assembly having plural clip devices connected with one another in a train by use of claws positioned at distal ends of openable arms, a fastening clip device connected with a final one of the clip devices, and a fitting connector, disposed on the fastening clip device, for mating with a shaft head in a direction transverse to a direction of the train of the clip devices at a distal end of an operating wire for operating the clip devices. The multiple clip applicator system includes a housing for containing the multiple clip assembly. There is a coupling device including a stage portion for introducing the multiple clip assembly from the housing, to enable coupling with a flexible sheath through which the operating wire extends. A receiving opening has a form slightly larger than a profile of the shaft head and a portion of the operating wire as viewed in the transverse direction, for receiving insertion of the shaft head and the portion of the operating wire to mate the shaft head with the fitting connector on the stage portion. The coupling device operates upon pull of the operating wire after mating the shaft head with the fitting connector, to enter the multiple clip assembly from the stage portion into the flexible sheath in a proximal direction thereof. A handle device moves the operating wire relative to the flexible sheath with stroke according to a length of the arms and a sequence of the train, to carry out treatment with each one of the clip devices in the multiple clip assembly.

The multiple clip assembly is a selected one of at least first and second multiple clip assemblies which are different in at least one of a length of the arms and a number of the clip devices in the train. The shaft head is a selected one of first and second shaft heads which are different in a shape in association with the first and second multiple clip assemblies. The receiving opening is selected from first and second receiving openings according to the first or second shaft head. Furthermore, a prevention device is positioned in at least one of the first and second receiving openings, for preventing the second shaft head from entry in the first receiving opening, and preventing the first shaft head from entry in the second receiving opening.

The handle device is a selected one of first and second handle devices which are different in stroke of the shaft head and the operating wire in association with respectively the first and second multiple clip assemblies. The coupling device is a selected one of first and second coupling devices having respectively the first and second receiving openings.

The shaft head includes a first hook projection, having a common shape between the first and second multiple clip assemblies, for engagement with the fitting connector. A second hook projection has a shape different between the first and second multiple clip assemblies.

The second hook projection is positioned on a proximal side from the first hook projection.

The second hook projection includes at least one peripheral groove of which a position, number or size is different between the first and second multiple clip assemblies. The prevention device includes an internal projection, formed with each of the first and second receiving openings, for insertion in the peripheral groove.

The second hook projection includes a coil associated with at least one of the first and second multiple clip assemblies, and disposed about a distal end of the operating wire. The receiving opening has a width for passing the coil.

In one preferred embodiment, the second hook projection is in a drum shape, and has a difference between the first and second multiple clip assemblies in at least one of a number, outer diameter and length thereof.

The at least first and second multiple clip assemblies include a multiple clip assembly of a standard size, a multiple clip assembly of a long size having the clip devices of which the arms are longer than the arms in the standard size, and a multiple clip assembly of a short size having the clip devices of which the arms are shorter than the arms in the standard size.

Also, a prevention device for preventing mismatch in a multiple clip applicator system is provided. The multiple clip applicator system includes a multiple clip assembly having plural clip devices and a fastening clip device, the clip devices being connected with one another in a train by use of claws positioned at distal ends of openable arms, the fastening clip device being connected with a final one of the clip devices. A handle device has a flexible sheath. A coupling device contains the multiple clip assembly and loading the flexible sheath with the multiple clip assembly. The multiple clip assembly is a selected one of at least first and second multiple clip assemblies which are different in at least one of a length of the arms and a number of the clip devices in the train, the coupling device is selected from first and second coupling devices according to the first or second multiple clip assembly, and the handle device is selected from first and second handle devices according to the first or second multiple clip assembly. The prevention device includes a shaft head, selected from first and second shaft heads which are different in a shape according to the first and second multiple clip assemblies, positioned on an operating wire in the flexible sheath. A receiving opening is selected from first and second receiving openings according to the first or second shaft head, formed in the coupling device, for receiving insertion of the shaft head, for mating with the fastening clip device. A prevention portion is positioned in at least one of the first and second receiving openings, for preventing the second shaft head from entry in the first receiving opening, and preventing the first shaft head from entry in the second receiving opening.

There are two or more parameters for determining the first and second shaft heads in forms distinct from one another.

The shaft head includes at least first and second hook projections disposed in an axial direction of the operating wire.

Accordingly, mismatch of a multiple clip assembly on a handle device is prevented.

### BRIEF DESCRIPTION OF THE DRAWINGS

The above objects and advantages of the present invention will become more apparent from the following detailed description when read in connection with the accompanying drawings, in which:
Fig. 1 is a perspective view illustrating a multiple clip applicator system;
Fig. 2 is a perspective view illustrating a multiple clip assembly;
Fig. 3 is a vertical section illustrating the multiple clip assembly;
Fig. 4 is an exploded perspective view illustrating a clip device and a tubular shell;
Fig. 5 is a vertical section illustrating the tubular shell;
Fig. 6 is a bottom plan illustrating the tubular shell;
Fig. 7 is a perspective view illustrating a fastening clip device and a shaft head;
Fig. 8 is a perspective view illustrating a clip package;
Fig. 9 is an exploded perspective view illustrating the clip package;
Fig. 10 is an exploded perspective view illustrating a housing;
Fig. 11 is an explanatory view in front elevation illustrating connection of a shaft head and a receiving opening;
Fig. 12 is a vertical section illustrating the clip package before use;
Fig. 13 is an explanatory view in development illustrating a sliding tube;
Fig. 14A is a vertical section illustrating the clip package where the multiple clip assembly is entered in a coupling device;
Fig. 14B is a vertical section illustrating access of the shaft head to the multiple clip assembly;
Fig. 15A is a vertical section illustrating the clip package where the shaft head is mated with the fastening clip device;
Fig. 15B is a vertical section illustrating the clip package where the mating of the shaft head is completed;
Fig. 16 is a vertical section illustrating the clip package in introduction of the fastening clip device in the flexible sheath;
Fig. 17A is a vertical section illustrating a state shortly before tissue clamping with a handle device;
Figs. 17B, 17C and 17D are vertical sections illustrating the tissue clamping with the handle device;
Fig. 18A is a side elevation illustrating the clip device and the tubular shell having different sizes;
Figs. 18B and 18C are side elevations illustrating clip devices and tubular shells according to the medium size and long size;
Fig. 19A is a vertical section illustrating a multiple clip assembly having the clip device and the tubular shell having the different sizes;
Figs. 19B and 19C are vertical sections illustrating multiple clip assemblies having the clip device and the tubular shell according to the medium size and long size;
Fig. 20A is an explanatory view in front elevation illustrating the shaft head and the receiving opening according to a type of the multiple clip assembly;
Figs. 20B and 20C are explanatory views in front elevation illustrating shaft heads and receiving openings according to the medium size and long size;
Fig. 21 is a table illustrating compatibility of a shaft head and a receiving opening;
Fig. 22A is an explanatory view in front elevation illustrating another preferred combination of a shaft head and a receiving opening;
Figs. 22B and 22C are explanatory views in front elevation illustrating combinations of a shaft head and a receiving opening according to the medium size and long size.

### DETAILED DESCRIPTION OF THE PREFERRED EMBODIMENT(S) OF THE PRESENT INVENTION

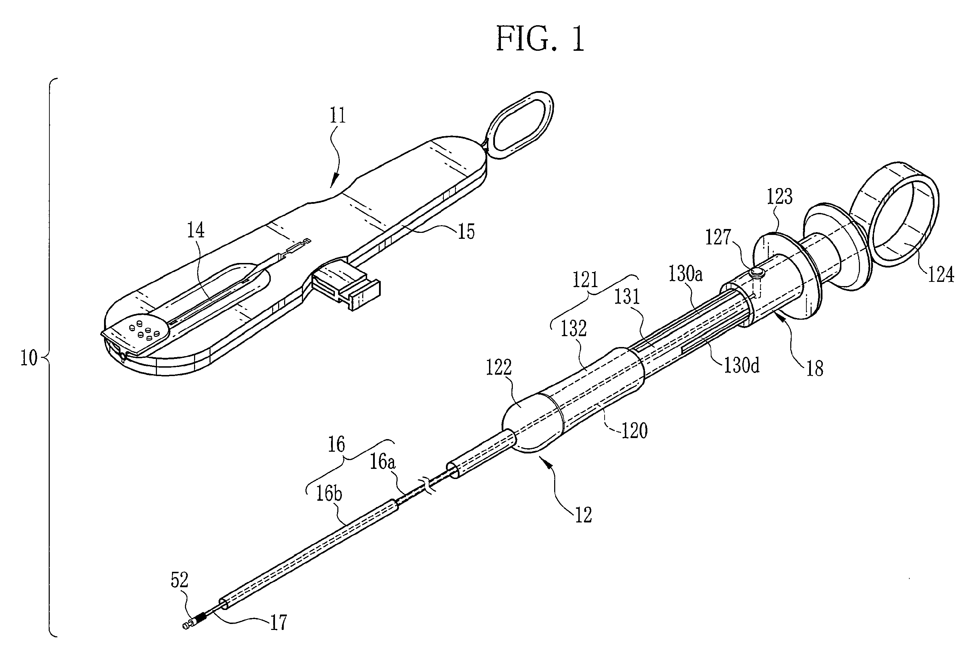

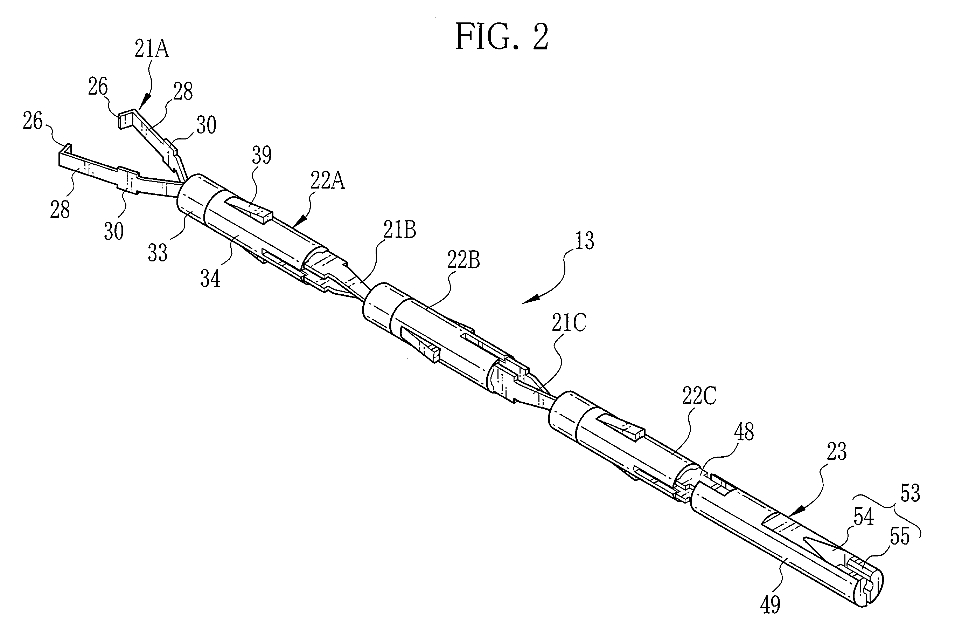

In Fig. 1, a multiple clip applicator system 10 or application apparatus includes a multiple clip package 11 or cartridge and a handle device 12. The multiple clip package 11 includes a housing 14 or barrel and a coupling device 15. A multiple clip assembly 13 or clip train of Fig. 2 is constituted by fastening plural clip devices. The housing 14 is a clip holder for holding and containing the multiple clip assembly 13. The coupling device 15 as a body of the multiple clip package 11 introduces the multiple clip assembly 13 from the housing 14 for loading of the multiple clip assembly 13 on the handle device 12. The handle device 12 includes a cylindrical flexible sheath 16, an operating wire 17 and a wire handle 18. The flexible sheath 16 is loaded with the multiple clip assembly 13 from the coupling device 15. The operating wire 17 is inserted through the flexible sheath 16, and coupled with a proximal end of the multiple clip assembly 13. The wire handle 18 moves the operating wire 17 relative to the flexible sheath 16, for operating clips of the multiple clip assembly 13 for tissue clamping.

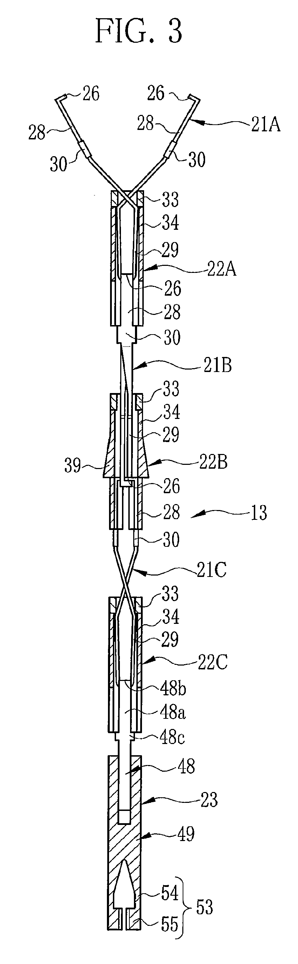

In Figs. 2 and 3, the multiple clip assembly 13 includes three hemostatic clip devices 21A, 21B and 21C, three tubular shells 22A, 22B and 22C or rings for retention, and a fastening clip device 23 or dummy clip device for retention. The clip devices 21A-21C are used for tissue clamping with an endoscope. The tubular shells 22A-22C are set about the clip devices 21A-21C. The fastening clip device 23 is connected with a proximal end of the clip device 21C. The clip devices 21A-21C are connected in series as a clip train. The tubular shells 22A-22C retain the connected state between the clip devices 21A-21C. The fastening clip device 23 is used for coupling with the operating wire 17.

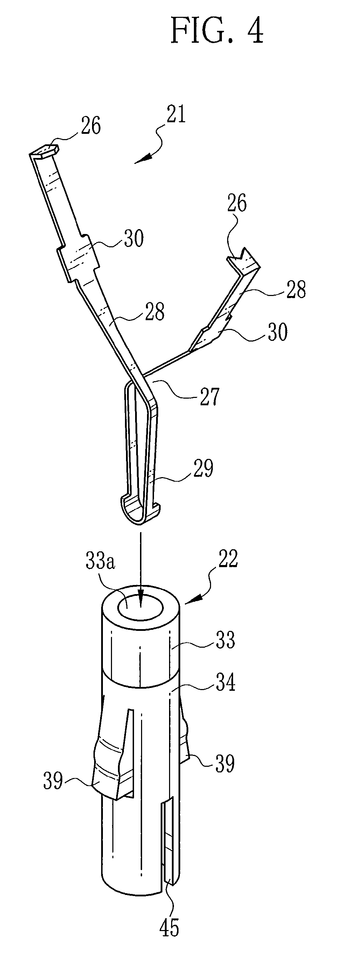

A hemostatic clip device 21 constitutes each of the clip devices 21A-21C in an equal form. A tubular shell 22 constitutes each of the tubular shells 22A-22C in an equal form. In Fig. 4, two claws 26 are formed with the clip device 21. At first, an elongate strip of a single plate is bent at an angle of 180 degrees. Segment portions extending at ends of the strip are crossed over one another, and opposed to one another by curving, to define the claws 26. The clip device 21 includes a crossover portion 27, arms 28 and a turn 29. The crossover portion 27 is defined by crossing the strip. The arms 28 are located at the free ends. The turn 29 is constituted by the closed end. Side projections 30 are formed on edges of the arms 28, are positioned at an intermediate point, and partially define portions with a greater width.

When the claws 26 of the clip device 21 are free without receiving external force, the claws 26 are open and away from one another. The claws 26 become meshed with one another and are in a closed position for tissue clamping when the arms 28 are deformed toward one another. The claws 26 are a V-shaped projection and notch in the combination for the purpose of clamping body tissue. An example of material for the clip device 21 is metal with biocompatibility, for example stainless steel SUS 631 for springs.

In Fig. 4, the tubular shell 22 has a cylindrical shape with a cavity for insertion of the clip device 21. The tubular shell 22 includes a push sleeve 33 and a support sleeve 34. The push sleeve 33 clamps and closes the claws 26. The support sleeve 34 retains a fastened state of the clip device 21. The push sleeve 33 of metal is fitted on the support sleeve 34 at its distal end. A narrow bore 33a is formed inside the push sleeve 33. A diameter of the narrow bore 33a is greater than a partial width of the clip device 21 at the crossover portion 27, and smaller than a width of the area of the side projections 30. An example of material for the push sleeve 33 is metal with biocompatibility, for example stainless steel SUS 304.

The push sleeve 33 is set in an initial position to cover the crossover portion 27. In Fig. 3, the arms 28 are in an open state when the push sleeve 33 is in its initial position. The push sleeve 33 with the narrow bore 33a pushes the arms 28 when moved to a position of contacting the side projections 30, to clamp and close the claws 26. The claws 26 exert force of clamping when the arms 28 are pushed toward one another.

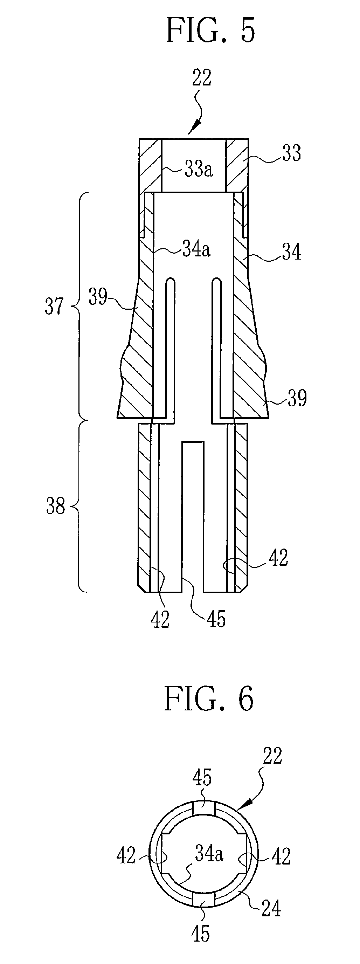

In Fig. 5, a bore 34a is formed through the support sleeve 34 for receiving the turn 29 and the arms 28 of the clip device 21. There are a first region 37 of deployment and a second region 38 of flexibility in the support sleeve 34. A preferable example of the material for the support sleeve 34 is polyphenylsulfone (PPSU or PPS) as resin having biocompatibility and flexibility, so as to deform resiliently according to steering of a guide tube of an endoscope.

Two fins 39 or skirt portions are formed on a peripheral surface of the support sleeve 34. The fins 39 extend with an increasing diameter in a direction from the distal end toward the proximal end of the support sleeve 34. The fins 39, when free without receiving external force, are deployed with their resiliency, and when depressed, become stowed by entry in the support sleeve 34. When depressed, the fins 39 clamp the clip device 21 contained in the support sleeve 34, and keep the clip device 21 positioned with the tubular shell 22 without an error. For tissue clamping with the handle device 12, the fins 39 become open upon advance through the distal opening of the flexible sheath 16, to keep the tubular shell 22 stopped at the distal opening of the flexible sheath 16.

In Figs. 5 and 6, two grooves 42 are formed in the bore 34a, located in the second region 38, opposed to one another, and positioned equally with the fins 39 in the rotational direction. The width of the grooves 42 is slightly greater than the maximum width of the arms 28 of the clip device 21, and smaller than the width of the area of the side projections 30. A distance between wall surfaces of the grooves 42 is equal to a sum of lengths of the claws 26 of the clip device 21 in the opening direction.

A length of the second region 38 is substantially equal to a distance at which the push sleeve 33 moves from the initial position of the clip device 21 toward its distal end to contact the side projections 30, namely a distance required for terminating clamping of the clip device 21. Also, the length of the second region 38 is substantially equal to a length of a portion of the clip device 21 from the claws 26 to an upper end of the side projections 30.

Two end channels 45 are formed in the second region 38, and disposed with a difference from the fins 39 with an angle of 90 degrees. The end channels 45 make the tubular shell 22 flexible. As an inner diameter of the second region 38 is enlarged because of the end channels 45, engagement of two of the clip devices 21 within the tubular shell 22 is facilitated in the course of assembly.

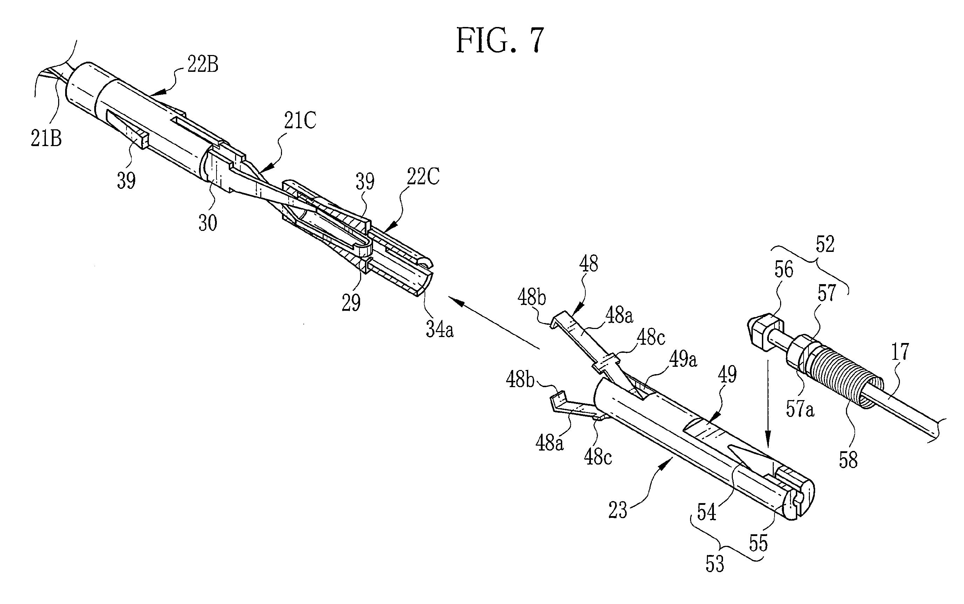

In Figs. 3 and 7, the fastening clip device 23 includes a clip 48 and a mating structure 49. The clip 48 may be produced from a material the same as that for the clip devices 21. A pair of arms 48a of the clip 48 are defined by bending a single elongate strip of a metal plate. The arms 48a are in an open state if free without external force. In a manner similar to the clip devices 21, claws 48b are formed with ends of the arms 48a. Side projections 48c are formed with intermediate portions of the arms 48a.

The mating structure 49 is a rod-shaped part formed from the same material as the support sleeve 34 in the tubular shell 22. A support recess 49a is formed in a distal end of the mating structure 49 for receiving the clip 48 and keeping the arms 48a movable for opening and closing. A fitting connector 53 or press-fit connector is disposed at a proximal end of the mating structure 49 for mating with the operating wire 17. A shaft head 52 for hooking or press-fit at a distal end of the operating wire 17 is coupled with the fitting connector 53. The fitting connector 53 includes a pair of cavity walls 54 with resiliency, and a pair of clamping walls 55. A press-fit opening is defined between the cavity walls 54 and the clamping walls 55. The cavity walls 54 are resilient in the radial direction of the mating structure 49. The clamping walls 55 are located at the end of the cavity walls 54.

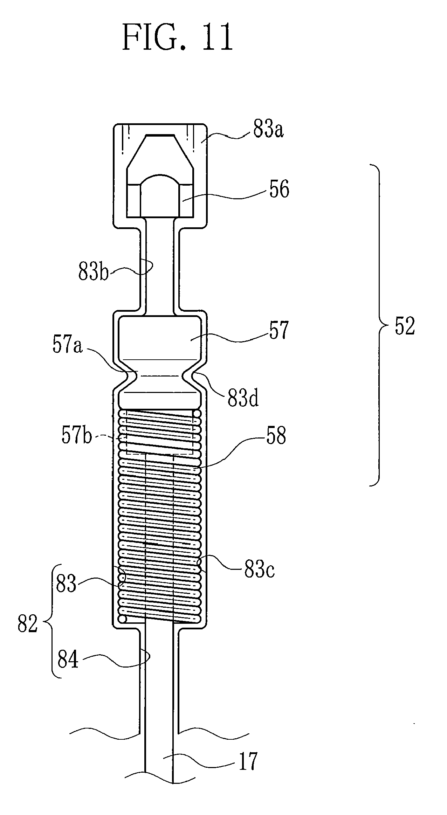

The shaft head 52 includes a first hook projection 56, and a second hook projection 57 disposed on a proximal side from the first hook projection 56. The first hook projection 56 has a shape of substantially a quadrangular pyramid, and is inserted between the cavity walls 54 by moving laterally or crosswise to the axial direction of the mating structure 49. The second hook projection 57 has a cylindrical shape, and contacts a proximal end of the clamping walls 55. In Fig. 11, the second hook projection 57 has a peripheral groove 57a and a coil 58 both for appropriate mating of the shaft head 52 with the fitting connector 53. The peripheral groove 57a is formed in an outer surface of the second hook projection 57. The coil 58 is positioned at a proximal end of the second hook projection 57. A small diameter portion 57b is disposed at the proximal end of the second hook projection 57. The coil 58 is fitted around the small diameter portion 57b, and disposed around a distal end of the operating wire 17. The coil 58 has a diameter equal to that of the second hook projection 57, and flexibility enough for bending together with the operating wire 17.

The multiple clip assembly 13 is constituted by connection of the clip devices 21A-21C, the tubular shells 22A-22C and the fastening clip device 23 in the following manner. In Fig. 3, the turn 29 of the clip device 21A is entered in the first region 37 of the tubular shell 22A through the narrow bore 33a. The clip device 21A is rotationally set with a difference of 90 degrees between an opening direction of the arms 28 and a deployment direction of the fins 39. The tubular shell 22A is set in the initial position where the push sleeve 33 covers the crossover portion 27 of the clip device 21A. In the clip device 21A inside the tubular shell 22A, a proximal end of the turn 29 protrudes into the second region 38. The arms 28 protrude externally from the tubular shell 22A and are in the open position.

In the second region 38 of the tubular shell 22A, the arms 28 of the clip device 21B are inserted from the proximal end of the bore 34a. As the clip device 21B is rotationally positioned with an angular difference of 90 degrees of the arms 28 from the clip device 21A in the first region 37, the arms 28 are inserted in the grooves 42. The clip device 21B entered in the second region 38 engages the claws 26 with the turn 29 of the clip device 21A.

The clip device 21B is kept connected with the clip device 21A because the tubular shell 22A prevents the claws 26 of the clip device 21B from shifting toward the open position. The clip devices 21A and 21B and the tubular shell 22A are axially regulated owing to the resiliency of the arms 28 of the clip device 21B inside the tubular shell 22A toward the open position and the contact of the side projections 30 of the clip device 21B with a proximal end of the tubular shell 22A. Also, the clip devices 21A and 21B and the tubular shell 22A are rotationally regulated owing to insertion of the arms 28 of the clip device 21B in the grooves 42 of the tubular shell 22A.

Fastening of the clip device 21C to the clip device 21B, retention with the tubular shell 22B for fastening, fastening of the fastening clip device 23 to the clip device 21C, and retention with the tubular shell 22C for fastening are the same as the conditions of the clip devices 21A and 21B and the tubular shell 22A.

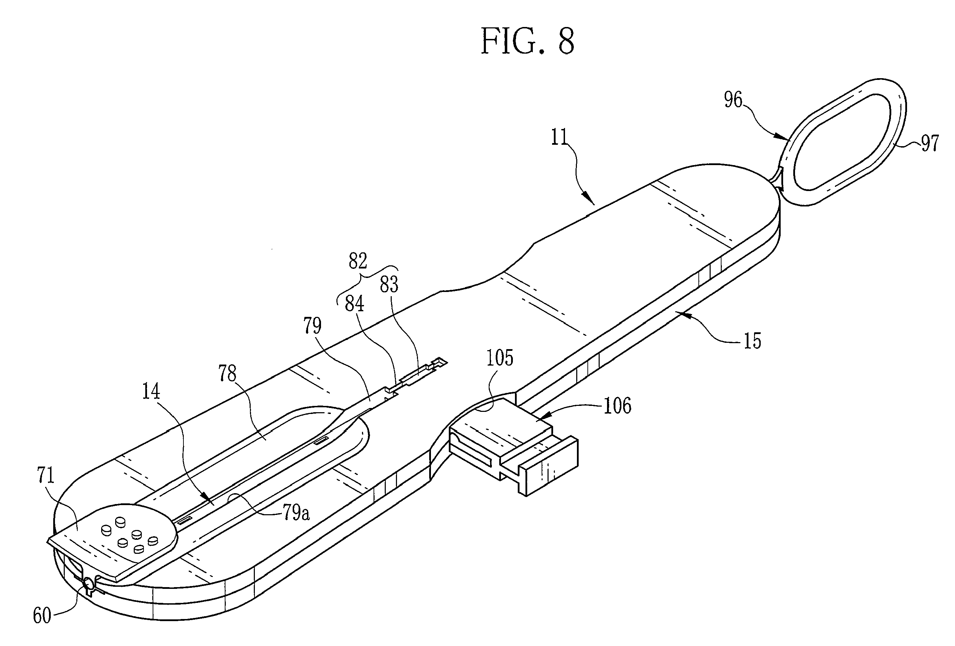

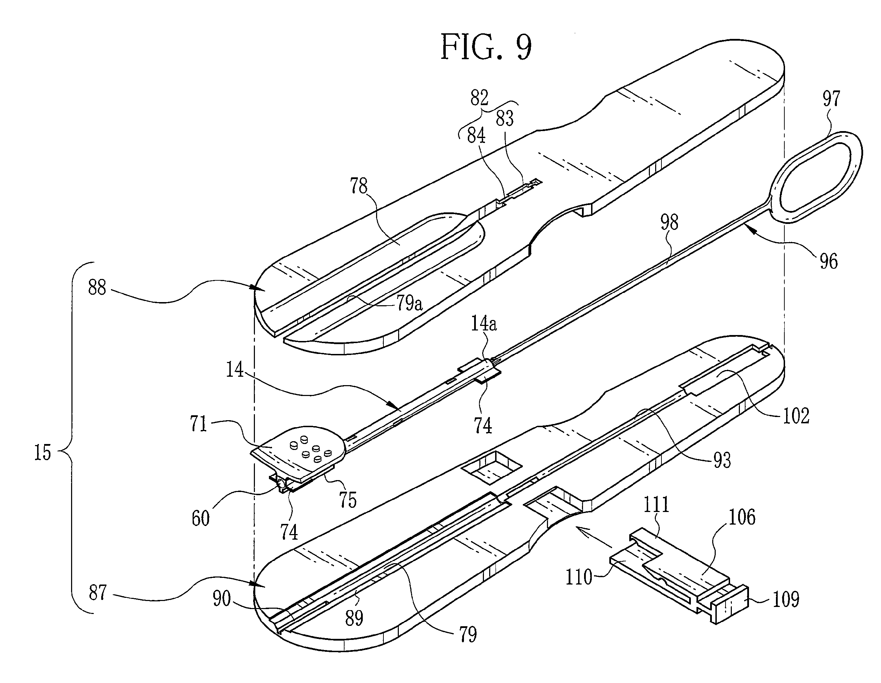

The multiple clip package 11 having the multiple clip assembly 13 according to the invention is described next. In Figs. 8 and 9, the multiple clip package 11 includes the housing 14 and the coupling device 15. The housing 14 contains the multiple clip assembly 13 in a state of keeping the fins 39 deployed in the clip devices 21A-21C. The coupling device 15 draws the multiple clip assembly 13 from the housing 14, depresses and stows the fins 39, couples the operating wire 17 with the fastening clip device 23, and loads the flexible sheath 16 with the multiple clip assembly 13.

The housing 14 is mounted on the coupling device 15 in a removable manner. A barrel cavity 60 is defined in the housing 14, and contains the multiple clip assembly 13. An outer diameter of the housing 14 is substantially equal to an outer diameter of the flexible sheath 16. An inner diameter of the barrel cavity 60 is substantially equal to the inner diameter of the flexible sheath 16.

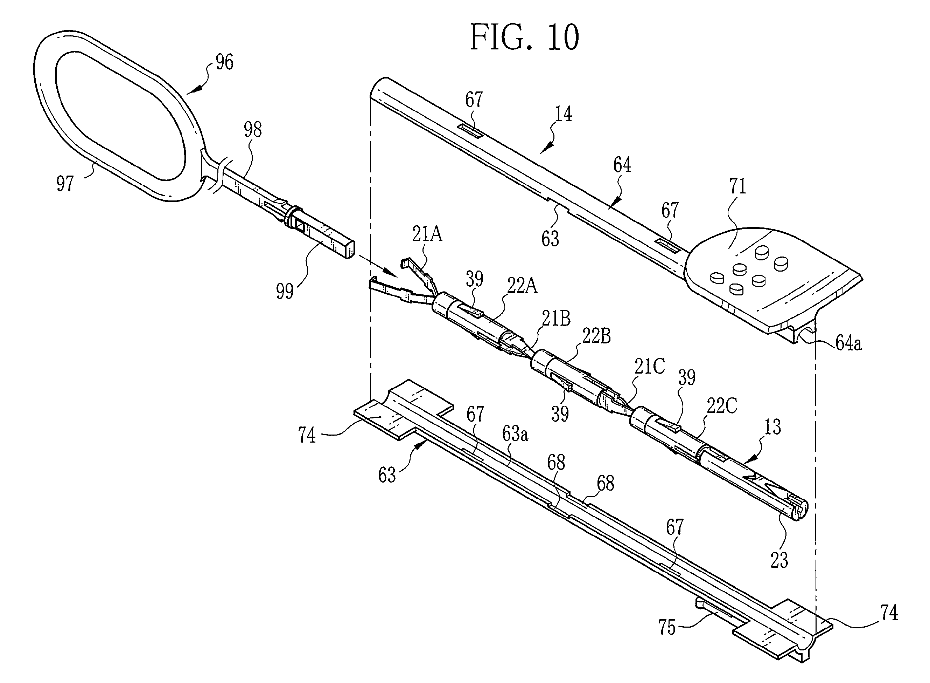

In Fig. 10, the housing 14 is constituted by a lower housing half 63 and an upper housing half 64 or barrel halves. An inner surface 63a of the lower housing half 63 and an inner surface 64a of the upper housing half 64 are semicylindrical, and combined to form the barrel cavity 60 of the cylindrical shape. The barrel cavity 60 is open at both of two ends, which include a first end for containing a distal end 14a with a distal opening or exit opening of the housing 14. The exit opening at the first end is open for advancing the multiple clip assembly 13 to the outside of the barrel cavity 60.

The multiple clip assembly 13 is contained in the barrel cavity 60 while the first clip device 21A is disposed at a distal end 14a of the housing 14 and the arms 28 of the first clip device 21A are open and closed laterally in the horizontal direction. The first clip device 21A is set in the closed position by the inside of the barrel cavity 60. As has been described heretofore, the clip devices 21A-21C are oriented with differences in the opening direction of the arms 28 with 90 degrees from one another. There is a difference between the claws 26 and the fins 39 in the opening direction with 90 degrees. Thus, the fins 39 of the tubular shells 22A and 22C are movable for deployment vertically in the depicted state. The fins 39 of the tubular shell 22B are movable for deployment horizontally in the depicted state.

Fin receiving slots 67 are formed in the upper and lower housing halves 63 and 64 and receive the fins 39 deployed from the tubular shells 22A and 22C. Recesses are formed in edge portions of the upper and lower housing halves 63 and 64, and define fin receiving slots 68 when joined, so as to receive the fins 39 deployed from the tubular shell 22B. This is effective in setting the fins 39 free from the pressing force toward the stowed position inside the housing 14. The force of recovery of the fins 39 toward the deployed position can be kept without lowering.

A grip tab 71 is formed on an upper surface of the upper housing half 64 and positioned at its proximal end. The grip tab 71 has a concave surface for touch of a user's finger, and used for pull of the housing 14 out of the coupling device 15.

The lower housing half 63 has plural ridges 74 and a key projection 75. The ridges 74 are four including two projecting from a distal end portion of the lower housing half 63 and two projecting from a proximal end portion of the same. The key projection 75 projects downwards. When the housing 14 is set on the coupling device 15, the ridges 74 and the key projection 75 become engaged with grooves or holes formed in the coupling device 15, and keep the housing 14 oriented suitably.

In Fig. 8, a concave surface 78 is formed in the coupling device 15, and disposed at its end with a reduced height. A stage groove 79 for housing reception is formed in a wall having the concave surface 78, has an inner diameter slightly greater than an outer diameter of the housing 14, and has an open upper side. The housing 14 is inserted in the stage groove 79. For the purpose of loading the flexible sheath 16 with the multiple clip assembly 13 from the coupling device 15, the housing 14 is removed from the stage groove 79 before inserting the flexible sheath 16 instead.

An elongated slot 79a is a cavity extending from the stage groove 79, and causes the housing 14 or the flexible sheath 16 to appear in the concave surface 78. The housing 14 or the flexible sheath 16 mounted in the stage groove 79 is kept with the coupling device 15 through the elongated slot 79a.

A receiving opening 82 is formed in the wall of the coupling device 15 and adjacent to the stage groove 79. The receiving opening 82 receives insertion of the operating wire 17 and the shaft head 52 after pulling of the multiple clip assembly 13 from the housing 14 into the coupling device 15, for mating the shaft head 52 with the mating structure 49 of the fastening clip device 23. The receiving opening 82 includes an access window 83 and a wire channel 84. The access window 83 receives insertion of the shaft head 52 in a direction perpendicular to the axial direction of the operating wire 17. The wire channel 84 receives insertion of the operating wire 17. The wire channel 84 extends to the stage groove 79 and also the access window 83.

In Fig. 11, a state of insertion of the operating wire 17 and the shaft head 52 in the receiving opening 82 is illustrated. The access window 83 has a shape defined for pass through of the shaft head 52 as viewed in the transverse direction of the press-fit and greater than the same with a small difference. The access window 83 includes a first subsection 83a, a second subsection 83b and a third subsection 83c. The first subsection 83a receives the first hook projection 56. The second subsection 83b receives a portion of the operating wire 17 between the first and second hook projections 56 and 57. The third subsection 83c receives the second hook projection 57 and the coil 58. An internal projection 83d projects from an edge of the third subsection 83c for fitting in the peripheral groove 57a of the second hook projection 57, by way of a prevention device or keying device or anti-mismating device. The size of the access window 83 is only 0.1 mm greater than the profile of the shaft head 52 in relation of the total length of the peripheral edge. A shaft head having only a small difference in the shape cannot be inserted in the access window 83.

In Fig. 9, the coupling device 15 is constituted by a lower casing 87 and an upper casing 88, each one of which has arcuate edges disposed at distal and proximal ends. The stage groove 79 and the elongated slot 79a are formed in respectively the lower casing 87 and the upper casing 88. A regulation groove 89 and a key way groove 90 are formed in a wall having the upper surface of the lower casing 87 and extend in an axial direction of the coupling device 15. The ridges 74 and the key projection 75 of the housing 14 are engaged with the regulation groove 89 and the key way groove 90.

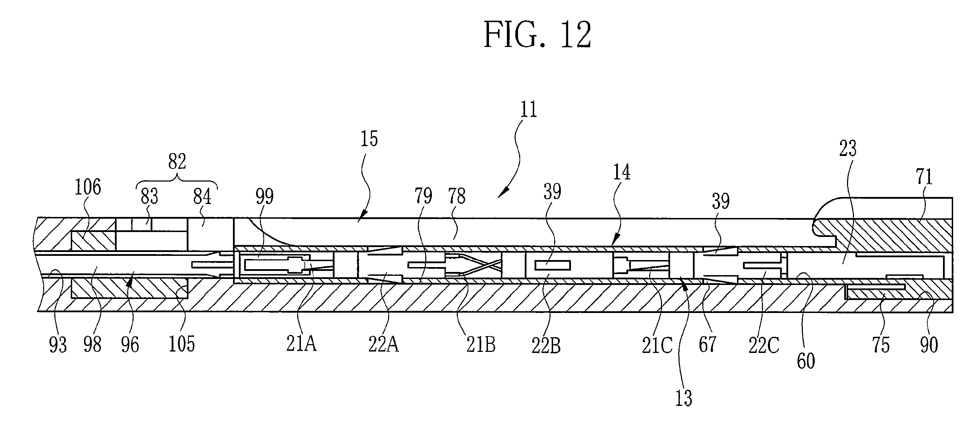

In Figs. 9 and 12, a fin bending channel 93 for closing is defined inside the coupling device 15 and positioned inwards from the stage groove 79 in a cylindrical shape. The fin bending channel 93 as a stage structure of the invention has a bore equal to that of the flexible sheath 16 and the barrel cavity 60, and is axially registered with the housing 14 inserted in the stage groove 79. An inner surface of the fin bending channel 93 is set in registration with that of the housing 14 and the flexible sheath 16 in the stage groove 79. The fin bending channel 93 contains the multiple clip assembly 13 entered from the barrel cavity 60, and depresses and stows the fins 39 with its inner surface. The receiving opening 82 described above is connected with the fin bending channel 93 inside the coupling device 15.

A pull rod structure 96 is assembled with the coupling device 15. The coupling device 15 introduces the multiple clip assembly 13 from the housing 14 into the fin bending channel 93 when guided by the pull rod structure 96. The pull rod structure 96 includes a pull tab 97, a shank 98 and an end connector 99. The pull tab 97 protrudes from the coupling device 15 in its longitudinal direction, and has an elliptic shape. The shank 98 extends from the pull tab 97. The end connector 99 is formed with an end of the shank 98 (See Fig. 12) . The shank 98 is inserted in the fin bending channel 93 with its length enough for penetration. The end connector 99 is inserted in the barrel cavity 60, and engaged with the first clip device 21A. When the pull tab 97 is pulled relative to the coupling device 15, the first clip device 21A of the multiple clip assembly 13 is pulled by the end connector 99 and entered in the fin bending channel 93 through the barrel cavity 60.

A release groove 102 is formed to extend horizontally from the fin bending channel 93. When the multiple clip assembly 13 is pulled into the fin bending channel 93 by the pull rod structure 96 to set the fitting connector 53 of the mating structure 49 under the receiving opening 82, the arms 28 of the clip 21A become open with its resiliency and disengaged from the end connector 99 by the release groove 102. Thus, the multiple clip assembly 13 is set in a stationary manner.

In Fig. 8, a slide channel 105 or auxiliary path is formed through a lateral wall of the coupling device 15, and extends in a direction perpendicular with the fin bending channel 93 under the receiving opening 82. The slide channel 105 extends in the transverse direction of the coupling device 15 toward its second lateral wall. There is a guide slider 106 inserted in the slide channel 105 in a slidable manner. The guide slider 106 operates when pressed in the slide channel 105 for connecting the fastening clip device 23 with the shaft head 52.

In Fig. 9, the guide slider 106 is one piece molded from a plastic material, and includes a button head 109, a lower slide plate 110 and an upper slide plate 111. The button head 109 is operable for depression into the slide channel 105. The slide plates 110 and 111 are opposed to one another, and extend from the button head 109 horizontally. When the multiple clip package 11 is unused, a main portion of the guide slider 106 protrudes laterally from the coupling device 15. See Fig. 8.

When the pull rod structure 96 pulls the multiple clip assembly 13 into the fin bending channel 93 to insert the flexible sheath 16 into the stage groove 79 in place of the housing 14 and insert the operating wire 17 and the shaft head 52 in the receiving opening 82, then the guide slider 106 is pushed into the slide channel 105. Then the upper and lower slide plates 110 and 111 of the guide slider 106 come to sandwich the mating structure 49 and the shaft head 52 in a vertical direction. The first hook projection 56 and the operating wire 17 are pressed into the press-fit opening between the cavity walls 54 and the clamping walls 55, to couple the multiple clip assembly 13 with the operating wire 17.

The handle device 12 is described next. In Fig. 1, the handle device 12 includes the flexible sheath 16, the operating wire 17 and the wire handle 18. An example of the flexible sheath 16 is a flexible coil sheath in which a wire of metal is tightly wound in a coiled form. The flexible sheath 16 includes an inner tube 16a for wire insertion and an outer sleeve 16b for clip setting. The inner tube 16a is connected with the wire handle 18. The outer sleeve 16b is disposed at a distal end as free end.

The inner tube 16a has an inner diameter sufficient for insertion of the operating wire 17, and constitutes a main part of the flexible sheath 16. The outer sleeve 16b has an inner diameter for disengaging the turn 29 of the clip device 21 from the claws 26 of a succeeding one of the clip devices 21. An inner diameter of the outer sleeve 16b is greater than a sum of a length of the claws 26 and a width of a portion of the turn 29 for engagement with the claws 26.

The operating wire 17 is a part of metal, and inserted through the flexible sheath 16 and the wire handle 18 movably back and forth. The shaft head 52 of Fig. 7 is secured to a distal end of the operating wire 17. When the operating wire 17 is pulled, a proximal end of the first hook projection 56 pushes the clamping walls 55 to transmit force of pulling to the multiple clip assembly 13. When the operating wire 17 rotates, a peripheral surface of the first hook projection 56 transmits rotations of the operating wire 17 to the cavity walls 54. Furthermore, when the operating wire 17 moves forwards, a distal end of the second hook projection 57 pushes a proximal end of the clamping walls 55, and transmits force of push to the multiple clip assembly 13.

The wire handle 18 includes a sheath handle 120, a slide mechanism 121, a regulating portion 122 and a sliding finger flange 123. The sheath handle 120 has a cylindrical shape. The slide mechanism 121 is mounted about the sheath handle 120 in a rotatable manner. The regulating portion 122 is a mechanism for regulating a stop position of the slide mechanism 121. The sliding finger flange 123 is mounted about the slide mechanism 121 and slidable in an axial direction. A thumb ring 124 is formed with a proximal end of the sheath handle 120. For example, a thumb of an operator or doctor is inserted in the thumb ring 124. His or her index finger and middle finger push the sliding finger flange 123 to slide the wire handle 18.

A through hole and a slide groove (not shown) are formed in the sheath handle 120. The through hole extends in the axial direction. The slide groove extends axially from the outer surface to the through hole. The operating wire 17 is inserted in the through hole. A guide pin 127 for sliding is fixed on the sliding finger flange 123, and inserted in the slide groove. A proximal end of the operating wire 17 is retained on the guide pin 127. When the sliding finger flange 123 is slid while guided by the guide pin 127 and the slide groove, the operating wire 17 moves relative to the flexible sheath 16.

The slide mechanism 121 has a sliding tube 131 and a guide barrel 132. Four guide grooves 130a, 130b, 130c and 130d are formed in the sliding tube 131, extend in the axial direction, and are positioned rotationally regularly. The guide barrel 132 rotates the sliding tube 131 about the sheath handle 120. When the guide barrel 132 is manually rotated, the slide groove of the sheath handle 120 is set on a selected one of the guide grooves 130a-130d. The guide grooves 130a-130d have different lengths, any one of which is smaller than the length of the slide groove. An amount of sliding of the sliding finger flange 123 is changed according to one of the guide grooves 130a-130d where the slide groove is set.

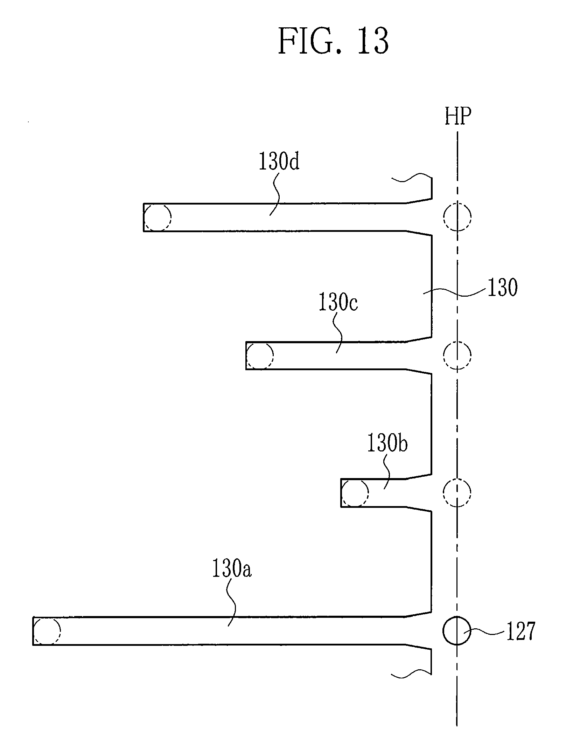

In Fig. 13, the sliding tube 131 is illustrated in a developed state. When the sliding finger flange 123 is slid and set at the proximal end of the sheath handle 120, the guide pin 127 is in a home position (HP) out of the guide grooves 130a-130d. The guide groove 130a has a greatest length, and is used for loading the flexible sheath 16 with the multiple clip assembly 13 from the coupling device 15, or for removing the fastening clip device 23 from the operating wire 17 after use. When the sliding finger flange 123 is slid for the guide pin 127 to contact an end of the guide groove 130a, the operating wire 17 and the shaft head 52 come to protrude from the distal opening of the flexible sheath 16.

The guide groove 130b with the smallest length is used for tissue clamping with the first clip device 21A. The moving amount of the operating wire 17 is small because the first clip device 21A is disposed near to the distal opening of the flexible sheath 16. The guide grooves 130c and 130d are used for tissue clamping with respectively the clip devices 21B and 21C owing to greater lengths than the guide groove 130b.

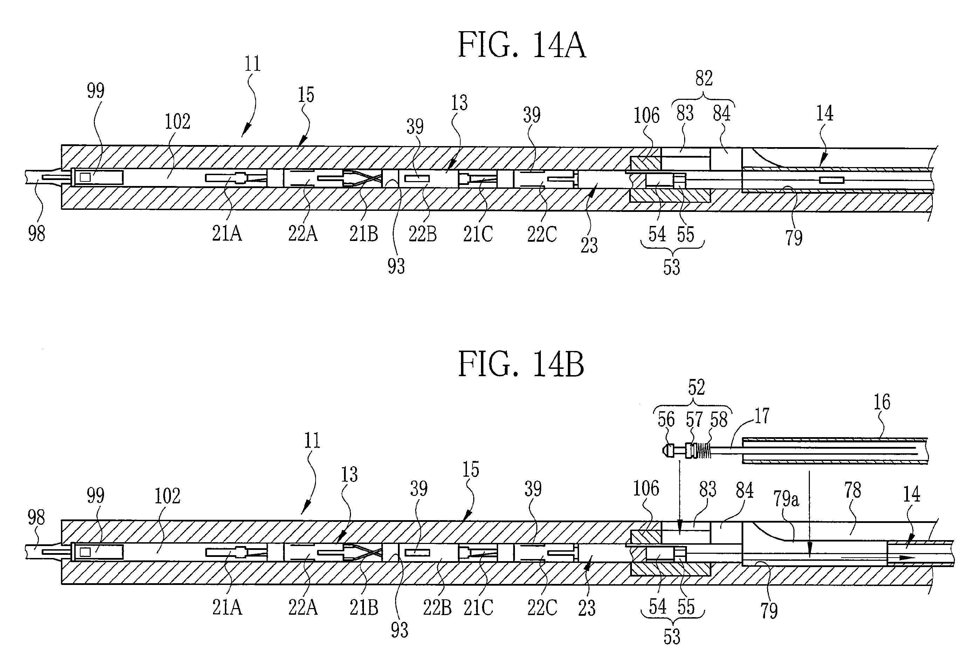

A method of loading of the multiple clip assembly 13 in the flexible sheath 16 from the multiple clip package 11 is described by referring to Figs. 14A-16. In Fig. 8, the housing 14 and the coupling device 15 are retained together by use of the concave surface 78 to pull the pull tab 97 from the coupling device 15. In Fig. 14A, the shank 98 is pulled out of the coupling device 15. The end connector 99 following the shank 98 moves into the fin bending channel 93. The multiple clip assembly 13 is pulled from the barrel cavity 60 into the fin bending channel 93 by the end connector 99.

In the multiple clip assembly 13 drawn in the fin bending channel 93, the fins 39 of the tubular shells 22A-22C are depressed and stowed by the inside of the fin bending channel 93. As the tubular shells 22A-22C are regulated by contact of their proximal end on the side projections 30 of one of the clip devices 21B and 21C, no error in positioning will occur between the clip devices 21A-21C and the tubular shells 22A-22C.

When the clip device 21A reaches the release groove 102, the arms 28 of the clip device 21A become released, to disengage the claws 26 from the end connector 99. The multiple clip assembly 13 is stopped in a position to set the fitting connector 53 of the mating structure 49 exactly at the access window 83 of the receiving opening 82. In Fig. 14B, the housing 14 is moved away from the stage groove 79 in an axial direction.

In Figs. 1 and 13, the guide groove 130a of the sliding tube 131 is positioned on the slide groove in the handle device 12. The sliding finger flange 123 is slid to a slide position where the guide pin 127 contacts an end of the guide groove 130a. Thus, the operating wire 17 and the shaft head 52 protrude from a distal opening of the flexible sheath 16.

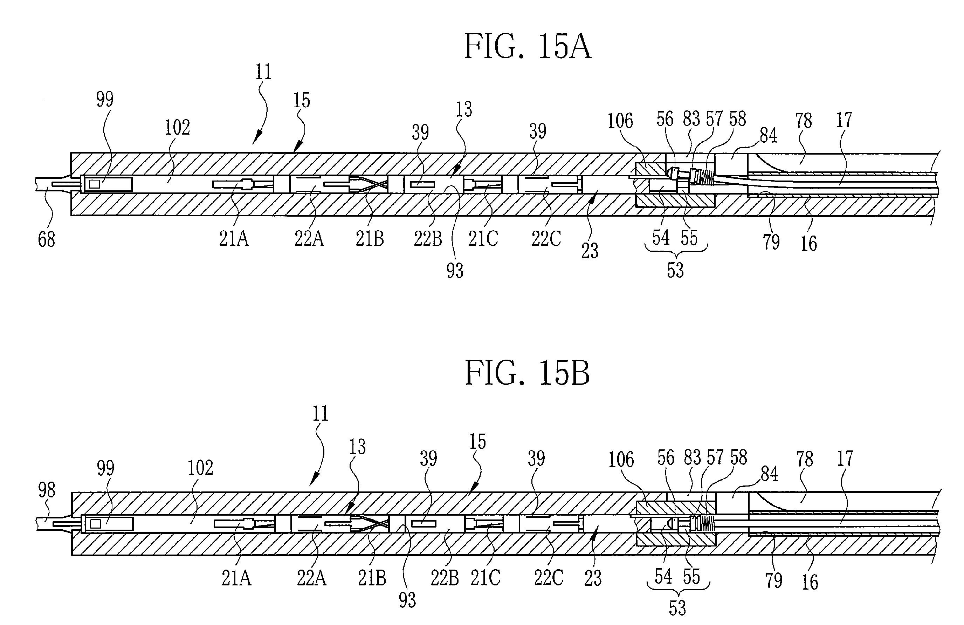

In Fig. 14B, the flexible sheath 16 is inserted in the stage groove 79 in place of the housing 14. The operating wire 17 and the shaft head 52 are inserted in the receiving opening 82 in a downward direction relative to the coupling device 15. After their insertion, the flexible sheath 16 and the coupling device 15 are retained together by use of the concave surface 78. In Fig. 15B, the shaft head 52 is mated by press-fit with the fitting connector 53.

In Fig. 11, the access window 83 of the receiving opening 82 is slightly larger than the profile of the shaft head 52 for its pass through. In the access window 83, it is impossible to insert another shaft head with a different shape from that of the shaft head 52. This is effective in preventing mismatch of the multiple clip assembly 13.

In Fig. 15B, the guide slider 106 is pushed into the slide channel 105. The upper and lower slide plates 110 and 111 on the guide slider 106 squeeze the shaft head 52 and the fitting connector 53 vertically. The first hook projection 56 and the operating wire 17 becomes fitted in the press-fit opening between the cavity walls 54 and the clamping walls 55. The second hook projection 57 contacts a proximal end of the mating structure 49. Finally, the operating wire 17 becomes coupled with the multiple clip assembly 13.

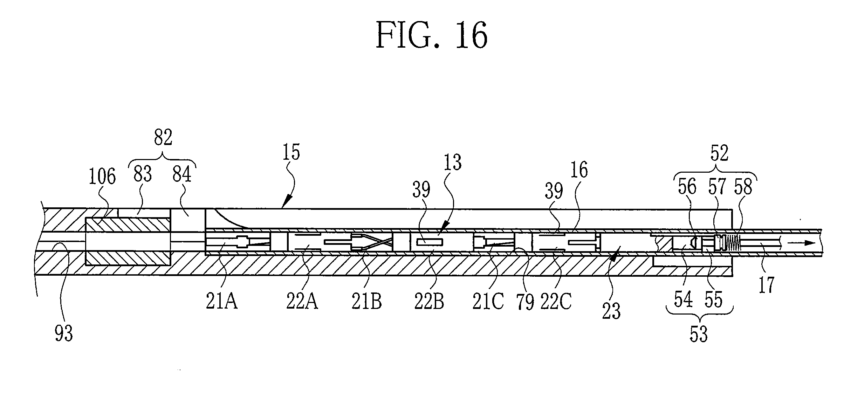

In Figs. 1 and 13, the sliding finger flange 123 is slid in the proximal direction to move the guide pin 127 to the home position HP from the end of the guide groove 130a. In Fig. 16, the operating wire 17 is pulled to introduce the multiple clip assembly 13 into the flexible sheath 16 by advancing its proximal end. During introduction of the multiple clip assembly 13, the inside of the flexible sheath 16 is in registration with the fin bending channel 93. The multiple clip assembly 13 can be moved only with a small resistance, because the fins 39 are depressed and stowed. The multiple clip assembly 13 can be loaded on the flexible sheath 16 without an error in positioning the clip devices 21A-21C and the tubular shells 22A-22C. The flexible sheath 16 is moved away from the flexible sheath 16 after loading of the multiple clip assembly 13.

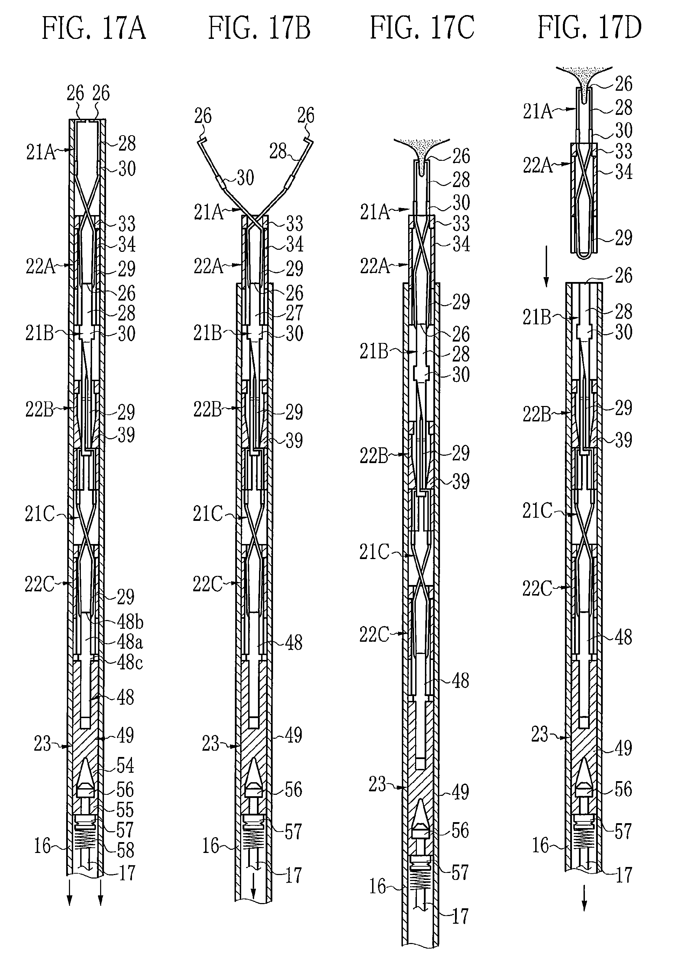

Tissue clamping by use of the multiple clip assembly 13 is described now. In Fig. 17A, the flexible sheath 16 having the outer sleeve 16b loaded with the multiple clip assembly 13 is inserted in an instrument channel in an endoscope entered in a body. A distal end of the flexible sheath 16 is advanced through a distal opening of the endoscope, and accesses affected tissue.

In Figs. 1 and 13, the guide groove 130b of the sliding tube 131 is positioned on the slide groove in the handle device 12. The sliding finger flange 123 is slid to a slide position where the guide pin 127 contacts an end of the guide groove 130b. In Fig. 17B, the first clip device 21A and the tubular shell 22B become protruded from the distal opening of the flexible sheath 16 by the operating wire 17. The arms 28 of the first clip device 21A become open by the resiliency. The fins 39 of the tubular shell 22A become deployed and engaged with the distal opening of the flexible sheath 16. Now the first clip device 21A is ready for use.

When the operating wire 17 moves forwards relative to the flexible sheath 16, friction occurs between the flexible sheath 16 and the tubular shells 22A-22C inside. However, the fins 39 in the stowed position keep the tubular shells 22A-22C engaged firmly with the clip devices 21A-21C. No offsetting occurs rotationally or in a forward or backward direction. The tubular shells 22A-22C will not move relative to the clip devices 21A-21C.

The entirety of the wire handle 18 is moved to press the claws 26 of the first clip device 21A in the open position against affected tissue. Then the sliding finger flange 123 is slid in the proximal direction to pull the operating wire 17 at a predetermined amount. In response, the clip devices 21A-21C are pulled equally in a state engaged serially from the fastening clip device 23.

As the fins 39 are deployed in the tubular shell 22A advanced from the distal opening of the flexible sheath 16 in the state of Figs. 17B and 17C, the clip device 21A is released from push with the fins 39. The tubular shell 22A is prevented from moving back into the flexible sheath 16 by the fins 39. In Fig. 17C, the clip device 21A is moved relative to the tubular shell 22A in the proximal direction by pull of the operating wire 17. When the push sleeve 33 is pressed to a position directly under the side projections 30 of the clip device 21A, the clip device 21A is closed entirely by the tubular shell 22A.

At the same time as the closing of the clip device 21A, an engaged portion between the clip devices 21A and 21B is moved out of the proximal end of the tubular shell 22A. The arms 28 of the clip device 21B become open to contact the inside of the flexible sheath 16 by their resiliency. An interval between the claws 26 becomes greater than a width of the turn 29 of the clip device 21A, to disengage the clip device 21A from the clip device 21B. In Fig. 17D, the entirety of the wire handle 18 is pulled to move the distal opening of the flexible sheath 16 back from the affected tissue. The clip device 21A and the tubular shell 22A are separated from the distal opening of the flexible sheath 16. Similarly, tissue clamping by use of the clip devices 21B and 21C is carried out.

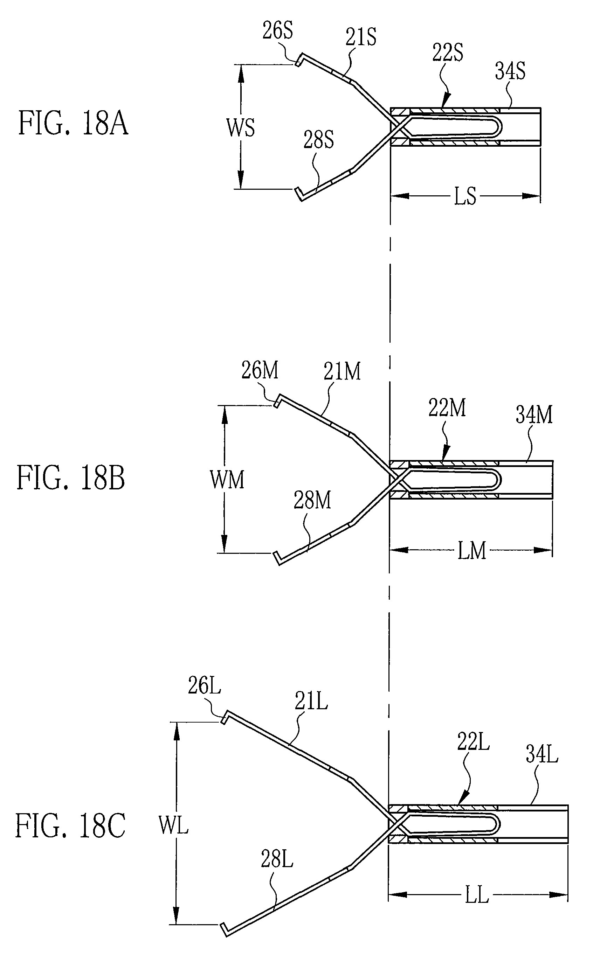

The multiple clip applicator system 10 of the invention has the clip devices 21 of three sizes which are short, medium and long sizes with different lengths of arms. Any one of the three can be selected for use with affected tissue by consideration of its size. A plurality of the tubular shells 22 are prepared in consideration of the sizes of the clip devices 21. In Fig. 18B, a hemostatic clip device 21M of a medium size is illustrated, and has arms 28M of a standard length. A second region in a support sleeve 34M has a size corresponding to the arms 28M in a tubular shell 22M for use with the clip device 21M. A total length of the tubular shell 22M is LM. The clip device 21M is structurally the same as the clip device 21 described above. The tubular shell 22M is structurally the same as the tubular shell 22.

In Fig. 18A, a hemostatic clip device 21S of a short size is illustrated. Arms 28S of the clip device 21S are shorter than those of the clip device 21M. An interval WS between claws 26S of the clip device 21S upon opening of the arms 28S is shorter than an interval WM between those of the clip device 21M, so that the clip device 21S is suitable for clamping of tissue of a small size. A tubular shell 22S is for use with the clip device 21S. A second region of a support sleeve 34S of the tubular shell 22S is longer than that of the support sleeve 34M in association with the arms 28S. A total length LS of the tubular shell 22S satisfies the condition LM > LS.

In Fig. 18C, a hemostatic clip device 21L of a long size is illustrated. Arms 28L of the clip device 21L are longer than those of the clip device 21M. An interval WL between claws 26L of the clip device 21L upon opening of the arms 28L is longer than an interval WM between those of the clip device 21M, so that the clip device 21L is suitable for clamping of tissue of a large size. A tubular shell 22L is for use with the clip device 21L. A second region of a support sleeve 34L of the tubular shell 22L is longer than that of the support sleeve 34M in association with the arms 28L. A total length LL of the tubular shell 22L satisfies the condition LL > LM > LS.

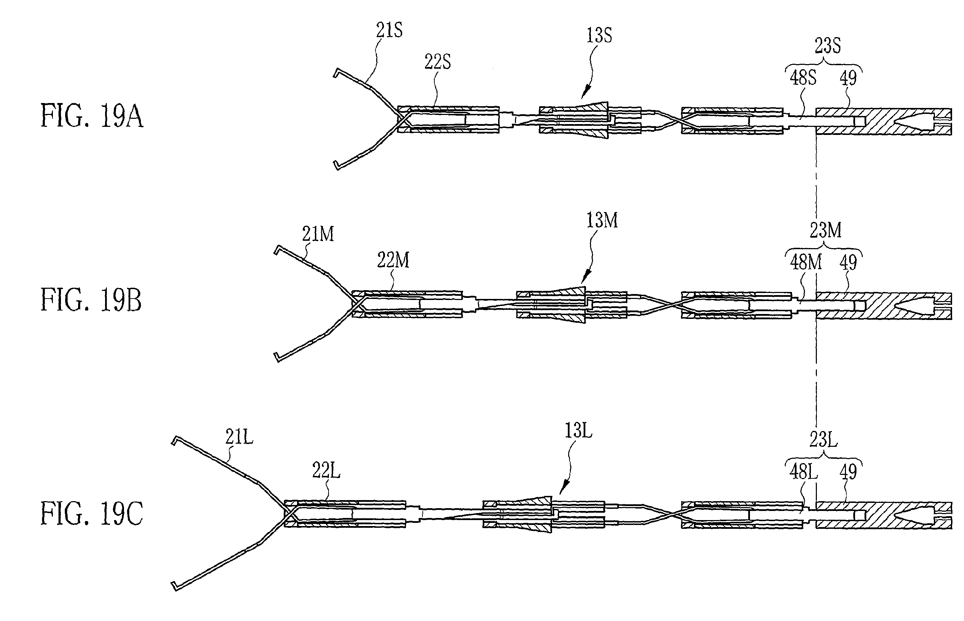

In Figs. 19A, 19B and 19C, the multiple clip applicator system 10 includes three multiple clip assemblies 13S, 13M and 13L or clip trains which respectively have the clip devices 21S, 21M and 21L and the tubular shells 22S, 22M and 22L. In combination, fastening clip devices 23S, 23M and 23L or dummy clip devices are used with respectively the multiple clip assemblies 13S, 13M and 13L. Clips 48S, 48M and 48L in the fastening clip devices 23S, 23M and 23L are different in the position for contact of the side projections 48c with a proximal end of the tubular shells 22S, 22M and 22L. However, the mating structure 49 is commonly included in each of the fastening clip devices 23S, 23M and 23L. Note the fastening clip device 23M is structurally the same as the fastening clip device 23.

In the multiple clip assemblies 13S, 13M and 13L, there are differences in the sizes between the clip devices 21S, 21M and 21L and the tubular shells 22S, 22M and 22L. The multiple clip assemblies 13S, 13M and 13L are different in the total length and the positions of the clip devices and tubular shells relative to the mating structure 49. Accordingly, the multiple clip applicator system 10 of the invention has three types of the multiple clip package 11 of which the housing 14 and the coupling device 15 are structurally specialized for types of the clip assemblies. For example, in the housing 14, the positions of the fin receiving slots 67 and 68 are different in compliance with the positions of the fins 39 of the tubular shells 22S, 22M and 22L. In the coupling device 15, the position of the release groove 102, the length of the pull rod structure 96 and the like are different in compliance with the total lengths of the multiple clip assemblies 13S, 13M and 13L.

The multiple clip assemblies 13S, 13M and 13L are different from one another in the stroke of the operating wire 17 for tissue clamping. Thus, the slide mechanism 121 is used in the multiple clip applicator system 10 with the guide grooves 130a-130d which have different lengths according to the multiple clip assemblies 13S, 13M and 13L. There are three types of the handle device 12 with which a movable range of the operating wire 17 is different.

If plural types of the multiple clip package 11 and plural types of the handle device 12 are combined for use, mismatch is likely to occur, for example, the multiple clip assembly 13L of the long size may be combined with the handle device 12 for the short size. An amount of the advance of the clip device 21 relative to the flexible sheath 16 may be too great or too small. Failure in operation may occur, for example, the arms 28 of the clip device 21 do not open suitably, or fastening may be released before tissue clamping.

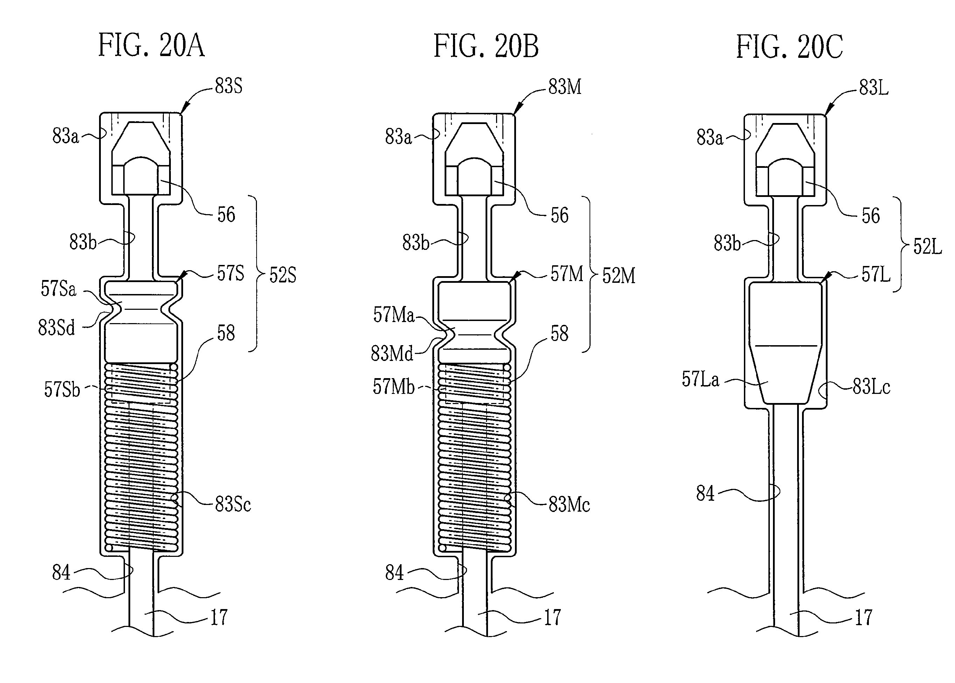

To solve the problem, the multiple clip applicator system 10 of one preferred embodiment of Figs. 20A-20C includes shaft heads 52S, 52M and 52L for hooking or press-fit and receiving openings 83S, 83M and 83L in combination in consideration of the multiple clip assemblies 13S, 13M and 13L. The mating structure 49 is a common element between the multiple clip assemblies 13S, 13M and 13L. So the first hook projection 56 is common between the shaft heads 52S, 52M and 52L. The first subsection 83a for insertion of the first hook projection 56 is the same between the receiving openings 83S, 83M and 83L.

In Fig. 20B, the shaft head 52M corresponding to the multiple clip assembly 13M of the medium size is illustrated. A peripheral groove 57Ma is formed in a second hook projection 57M and near to its proximal end. A small diameter portion 57Mb is formed at a proximal end of the second hook projection 57M. The coil 58 is fitted on the small diameter portion 57Mb. The receiving opening 83M corresponding to the multiple clip assembly 13M of the medium size has a third subsection 83Mc for insertion. The third subsection 83Mc has a shape near to that of the second hook projection 57M and the coil 58. Two internal projections 83Md are formed with edges of the third subsection 83Mc for insertion into the peripheral groove 57Ma. Note that the shaft head 52M and the receiving opening 83M are structurally the same as the shaft head 52 and the receiving opening 82 with the access window 83.

In Fig. 20A, the shaft head 52S corresponding to the multiple clip assembly 13S of the short size is illustrated. A peripheral groove 57Sa is formed in a second hook projection 57S structurally the same as the second hook projection 57M, and near to its proximal end. A small diameter portion 57Sb is formed at a proximal end of the second hook projection 57S. The coil 58 is fitted on the small diameter portion 57Sb. The receiving opening 83S corresponding to the multiple clip assembly 13S of the short size has a third subsection 83Sc for insertion. The third subsection 83Sc has a shape near to that of the second hook projection 57S and the coil 58. Two internal projections 83Sd are formed with edges of the third subsection 83Sc for insertion into the peripheral groove 57Sa, by way of a prevention device or keying device or anti-mismating device.

In Fig. 20C, the shaft head 52L corresponding to the multiple clip assembly 13L of the long size is cylindrical, and has a diameter equal to that of the second hook projections 57S and 57M. No peripheral groove is formed in the shaft head 52L. A second hook projection 57L has a proximal end where an inclined portion 57La is formed to have a decreasing diameter in a proximal direction. A total length of the second hook projection 57L is equal to that of the second hook projections 57S and 57M inclusive of the small diameter portions 57Sb and 57Mb. In the receiving opening 83L corresponding to the multiple clip assembly 13L of the long size, a third subsection 83Lc for insertion has a form near to the profile of the second hook projection 57L and enough to cover the same. The third subsection 83Lc is shorter than the third subsections 83Sc and 83Mc as the shaft head 52L is not provided with the coil 58.

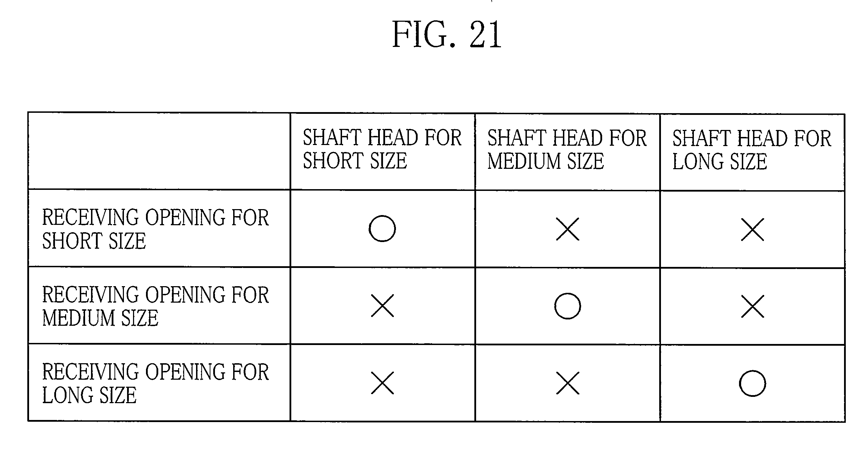

In Fig. 21, compatibility of the shaft heads 52S, 52M and 52L to the receiving openings 83S, 83M and 83L is indicated. A symbol of o denotes a matched status. A symbol of x denotes an unmatched status. The shaft heads 52S and 52M cannot be inserted in the third subsection 83Lc for the long size because of having the coil 58. The shaft head 52L cannot be inserted in any one of the third subsections 83Sc and 83Mc because the second hook projection 57L interferes with the internal projections 83Sd and 83Md. The shaft heads 52S and 52M are different from one another in relation to the positions of the peripheral grooves 57Sa and 57Ma and the internal projections 83Sd and 83Md. Even when one of the peripheral grooves is shifted for pass through of one of the internal projections, the first hook projection 56 operates for interference. Pass through of those with the receiving openings 83S and 83M is impossible. Thus, mismatch of the multiple clip assembly 13 on the handle device 12 can be prevented.

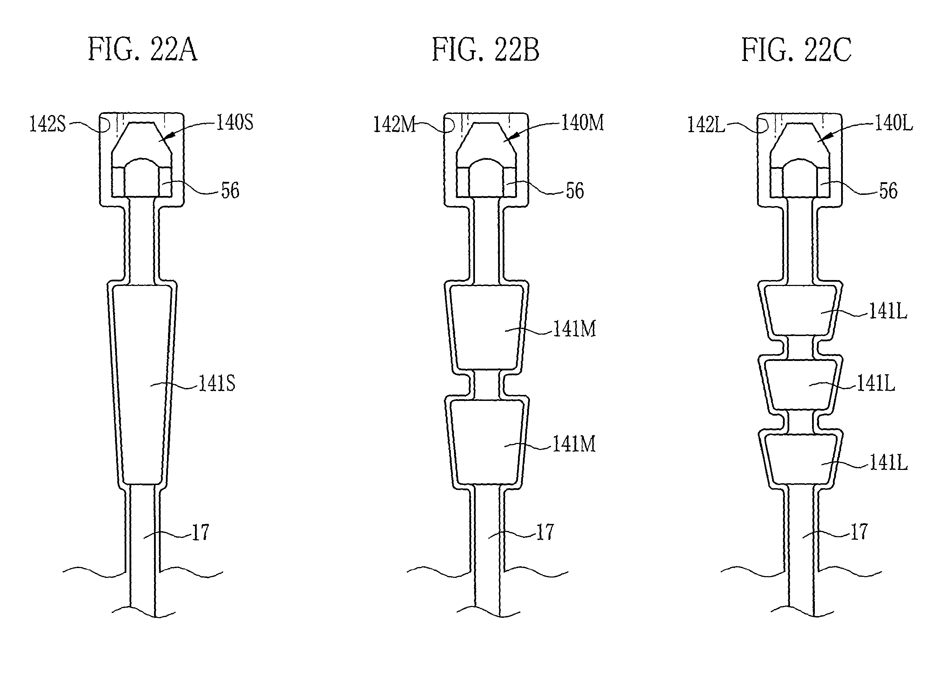

In the above embodiments, peripheral grooves in the second shaft head and internal projections in the receiving opening are changed for types of the clip assemblies. Furthermore, it is possible to change the numbers of the peripheral grooves and internal projections or their size. Also, the number, width and length of the second shaft head may be changed. In Figs. 22A, 22B and 22C, a second hook projection 141S in a shaft head 140S for the short size has a long and thin shape. Also, a shaft head 140M for the medium size has two second hook projections 141M. A shaft head 140L for the long size has three second hook projections 141L. The second hook projections 141M and 141L have forms with a greater thickness and smaller length than that of the shaft head 140S. Receiving openings 142S, 142M and 142L are defined for insertion of respectively the shaft heads 140S, 140M and 140L, and have a slightly greater size than matched shaft heads. Thus, mismatch can be prevented in a manner similar to the above embodiments. The number of parameters of structures of shaft heads in different forms may be equal to or more than the number of types of the clip assemblies.

In the above embodiment, the types of the clip assemblies are different according to different sizes of the clip devices. However, forms of the shaft head and the receiving opening can be changed according to types of the clip assemblies being different in the number of serially connected clip devices therein. Various modifications are possible for the clip device package and multiple clip applicator system according to the invention.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A clip package comprising:
a multiple clip assembly (13) having plural clip devices (21, 21A-21C, 21S, 21M, 21L) connected with one another in a train by use of claws (26) positioned at distal ends of openable arms (28), a fastening clip device (23) connected with a final one of said clip devices, and a fitting connector (53), disposed on said fastening clip device, for mating with a shaft head (52, 52S, 52M, 52L, 140, 140S, 140M, 140L) in a direction transverse to a direction of said train of said clip devices at a distal end of an operating wire (17) for operating said clip devices;
a housing (14) for containing said multiple clip assembly;
a coupling device (15), including:
a stage portion (79) for introducing said multiple clip assembly from said housing, to enable coupling with a flexible sheath through which said operating wire extends;
a receiving opening (82, 83S, 83M, 83L, 142S, 142M, 142L), having a form slightly larger than a profile of said shaft head and a portion of said operating wire as viewed in said transverse direction, for receiving insertion of said shaft head and said portion of said operating wire to mate said shaft head with said fitting connector on said stage portion;
wherein said coupling device operates upon pull of said operating wire after mating said shaft head with said fitting connector, to enter said multiple clip assembly from said stage portion into said flexible sheath in a proximal direction thereof.

2. A clip package as defined in claim 1, wherein said multiple clip assembly is a selected one of at least first and second multiple clip assemblies which are different in at least one of a length of said arms and a number of said clip devices in said train;
said shaft head is a selected one of first and second shaft heads which are different in a shape in association with said first and second multiple clip assemblies;
said receiving opening is selected from first and second receiving openings according to said first or second shaft head;
further comprising a prevention device, positioned in at least one of said first and second receiving openings, for preventing said second shaft head from entry in said first receiving opening, and preventing said first shaft head from entry in said second receiving opening.

3. A multiple clip applicator system, including a multiple clip assembly (13) having plural clip devices (21, 21A-21C, 21S, 21M, 21L) connected with one another in a train by use of claws (26) positioned at distal ends of openable arms (28), a fastening clip device (23) connected with a final one of said clip devices, and a fitting connector (53), disposed on said fastening clip device, for mating with a shaft head (52, 52S, 52M, 52L, 140, 140S, 140M, 140L) in a direction transverse to a direction of said train of said clip devices at a distal end of an operating wire (17) for operating said clip devices, said multiple clip applicator system comprising:
a housing (14) for containing said multiple clip assembly;
a coupling device (15), including:
a stage portion (79) for introducing said multiple clip assembly from said housing, to enable coupling with a flexible sheath (16) through which said operating wire extends;
a receiving opening (82, 83S, 83M, 83L, 142S, 142M, 142L), having a form slightly larger than a profile of said shaft head and a portion of said operating wire as viewed in said transverse direction, for receiving insertion of said shaft head and said portion of said operating wire to mate said shaft head with said fitting connector on said stage portion;
wherein said coupling device operates upon pull of said operating wire after mating said shaft head with said fitting connector, to enter said multiple clip assembly from said stage portion into said flexible sheath in a proximal direction thereof;
a handle device (12) for moving said operating wire relative to said flexible sheath with stroke according to a length of said arms and a sequence of said train, to carry out treatment with each one of said clip devices in said multiple clip assembly.

4. A multiple clip applicator system as defined in claim 3, wherein said multiple clip assembly is a selected one of at least first and second multiple clip assemblies which are different in at least one of a length of said arms and a number of said clip devices in said train;
said shaft head is a selected one of first and second shaft heads which are different in a shape in association with said first and second multiple clip assemblies;
said receiving opening is selected from first and second receiving openings according to said first or second shaft head;
further comprising a prevention device, positioned in at least one of said first and second receiving openings, for preventing said second shaft head from entry in said first receiving opening, and preventing said first shaft head from entry in said second receiving opening.

5. A multiple clip applicator system as defined in claim 4, wherein said handle device is a selected one of first and second handle devices which are different in stroke of said shaft head and said operating wire in association with respectively said first and second multiple clip assemblies;
said coupling device is a selected one of first and second coupling devices having respectively said first and second receiving openings.

6. A multiple clip applicator system as defined in claim 5, wherein said shaft head includes:
a first hook projection, having a common shape between said first and second multiple clip assemblies, for engagement with said fitting connector;
a second hook projection having a shape different between said first and second multiple clip assemblies.

7. A multiple clip applicator system as defined in claim 6, wherein said second hook projection is positioned on a proximal side from said first hook projection.

8. A multiple clip applicator system as defined in claim 6, wherein said second hook projection includes at least one peripheral groove of which a position, number or size is different between said first and second multiple clip assemblies;
said prevention device includes an internal projection, formed with each of said first and second receiving openings, for insertion in said peripheral groove.

9. A multiple clip applicator system as defined in claim 6, wherein said second hook projection includes a coil associated with at least one of said first and second multiple clip assemblies, and disposed about a distal end of said operating wire;
said receiving opening has a width for passing said coil.

10. A multiple clip applicator system as defined in claim 6, wherein said second hook projection is in a drum shape, and has a difference between said first and second multiple clip assemblies in at least one of a number, outer diameter and length thereof.

11. A multiple clip applicator system as defined in claim 6, wherein said at least first and second multiple clip assemblies include:
a multiple clip assembly of a standard size;
a multiple clip assembly of a long size having said clip devices of which said arms are longer than said arms in said standard size;
a multiple clip assembly of a short size having said clip devices of which said arms are shorter than said arms in said standard size.

12. A prevention device for preventing mismatch in a multiple clip applicator system;
said multiple clip applicator system including:
a multiple clip assembly (13) having plural clip devices (21, 21A-21C, 21S, 21M, 21L) and a fastening clip device (23), said clip devices being connected with one another in a train by use of claws (26) positioned at distal ends of openable arms (28), said fastening clip device being connected with a final one of said clip devices;
a handle device (12) having a flexible sheath (16);
a coupling device (15) for containing said multiple clip assembly and loading said flexible sheath with said multiple clip assembly;
wherein said multiple clip assembly is a selected one of at least first and second multiple clip assemblies which are different in at least one of a length of said arms and a number of said clip devices in said train, said coupling device is selected from first and second coupling devices according to said first or second multiple clip assembly, and said handle device is selected from first and second handle devices according to said first or second multiple clip assembly;
said prevention device comprising:
a shaft head (52, 52S, 52M, 52L, 140, 140S, 140M, 140L), selected from first and second shaft heads which are different in a shape according to said first and second multiple clip assemblies, positioned on an operating wire (17) in said flexible sheath;
a receiving opening (82, 83S, 83M, 83L, 142S, 142M, 142L), selected from first and second receiving openings according to said first or second shaft head, formed in said coupling device, for receiving insertion of said shaft head, for mating with said fastening clip device;
a prevention portion (83d, 83Sd, 83Md, 83Lc), positioned in at least one of said first and second receiving openings, for preventing said second shaft head from entry in said first receiving opening, and preventing said first shaft head from entry in said second receiving opening.

13. A prevention device as defined in claim 12, wherein there are two or more parameters for determining said first and second shaft heads in forms distinct from one another.

14. A prevention device as defined in claim 13, wherein said shaft head includes at least first and second hook projections disposed in an axial direction of said operating wire.
